# EUROPÄISCHE PATENTANMELDUNG

(11) **EP 0 623 820 A2**
(43) Veröffentlichungstag der Anmeldung: **09.11.1994**
(21) Anmeldenummer: 94102116.4
(22) Anmeldetag: 11.02.1994
(51) Int. Cl.: G01N 33/00, G01N 33/44, G01N 21/90

(54) **Verfahren zur Bestimmung von Kontaminationen in Mehrwegflaschen**

(30) Priorität: 05.03.1993 DE 4306833
(71) Anmelder: KHS Maschinen- und Anlagenbau Aktiengesellschaft, D-44143 Dortmund (DE)
(72) Erfinder: Heidrich, Karl Dr., D-45355 Essen (DE)

(57) **Zusammenfassung**

Die Erfindung bezieht sich auf ein Verfahren zur Bestimmung von kontaminierten Mehrwegflaschen mittels verschiedenartiger Analysatoren in einem Probenahmesystem. Hierbei soll eine einwandfreie Analyse der in wiederverwendbaren Getränkeflaschen befindlichen Reststoffe vorgenommen werden. Hierzu wird vorgeschlagen, daß ein erstes Probenahmesystem angeordnet ist, dessen zugeführte Proben einer Stickstoffoxyd-Analyse unterworfen werden und ein zweites Probenahmesystem vorgesehen ist, dessen Proben einer Photoionisationsanalyse und/oder Flammionisationsanalyse unterworfen und die vorgeprüften Flaschen dann einer Massenspektrometeranalyse unterworfen werden.

## Beschreibung

Die Erfindung bezieht sich auf ein Verfahren zur Bestimmung von kontaminierten Mehrwegflaschen oder Behältern mittels verschiedenartiger Analysatoren in einem Probenahmesystem.

Die Verwendung von Detektoren wie PID oder FID für die Analyse von verschiedenen Stoffen ist bekannt. Der Einsatz eines Analysatortyps führt insbesondere bei fortgeschrittener Vergärung von Produkten zu Fehlausschleusungen. Der größte Teil der rücklaufenden Mehrwegflaschen zeigt diesen Zustand der Vergärung. Die davon gewonnenen Meßergebnisse sind vergleichbar mit denen schwacher Kontaminate, woraus folgt, daß entweder schwache Kontaminate passieren oder der größte Teil der damit belasteten Flaschen ausgeschleust werden muß.

Aufgabe der Erfindung ist es, eine einwandfreie Analyse der in wiederverwendbaren Getränkeflaschen befindlichen Reststoffe vornehmen und je nach vorhandener Kontamination eine schnelle und zuverlässige Vorausscheidung solcher Flaschen vornehmen zu können, die dann gegebenenfalls erneut überprüft werden können.

Diese der Erfindung zugrunde liegende Aufgabe wird bei einem Verfahren der eingangs genannten Art dadurch gelöst, daß ein erstes Probenahmesystem angeordnet ist, dessen zugeführte Proben einer Stickstoffoxydanalyse unterworfen werden und ein zweites Probenahmesystem vorgesehen ist, dessen Proben einer Fotoionisationsanalyse und/oder Flammionisationsanalyse unterworfen und die vorgeprüften Flaschen dann einer Massenspektrometeranalyse unterworfen werden.

Dabei hat es sich in eigenständiger Ausbildung der Erfindung als zweckmäßig erwiesen, daß die zugeführten Flaschen unmittelbar vor der eigentlichen Hauptanalyse einer Ein- oder mehrfachen Voranalyse unterworfen, die dabei als fehlerhaft oder ungeeignet erkannten Flaschen aussortiert und die ordnungsgemäßen Flaschen einer weiteren Analyse unterworfen werden.

Ferner wird in selbständiger Ausgestaltung des Verfahrens gemäß Oberbegriff des Hauptanspruches vorgeschlagen, daß eine erste Analyse vorgenommen wird, bei welcher die Flaschen ermittelt und ausgeschleust werden, in denen eine andere als die zur Füllung vorgegebene Flüssigkeit enthalten war und diese Flaschen erneut zugeführt werden, wenn die in diesen analysierte Flüssigkeit zur Abfüllung gelangt.

Mit dem vorgeschlagenen Verfahren können praktisch verschiedenartige Analysen vorgenommen werden, wobei insbesondere Voranalysen möglich sind, bei denen beispielsweise Urin oder andere Kontaminate festgestellt und eine entsprechende Ausschleusung der Flaschen bereits vor der eigentlichen Hauptanalyse vorgenommen werden kann. Bei dem in Anspruch 1 eingesetzten Stickstoffoxydanalyseverfahren kann auf einfache Weise der praktisch in jedem Urin vorhandene Stickstoff beispielsweise durch ein Chemie-LumineszenzVerfahren analysiert werden. Hierbei handelt es sich um das sogenannte kalte Leuchten von Körpern oder Teilchen ohne Temperaturerhöhung durch chemische Vorgänge, bei denen Photonen freigesetzt werden. Bei dem Verfahren wird dem vorhandenen Ammoniak in einer katalytischen Oxidationszelle Wärme zugeführt, was dann zu Stickstoffoxyd und Wasser reagiert. Nach der Reaktion gelangt das Stickstoffoxyd in die Mischkammer, in der über einen Ozongenerator Ozon zugeführt wird. Bei der folgenden chemische Reaktion entstehen Sauerstoff, angeregtes Stickstoff-Dioxyd sowie Photonen. Die Photonen werden aus dem Chemie-Lumineszenz-Effekte emittiert. Zwischen der Misch- und Reaktionskammer und dem eigentlichen Multiplier, der die Auswertung vornimmt, wird das Lichtsignal mit einer Frequenz von beispielsweise 87 Hz in Einzelsignale zerlegt. Um kleinste Signale noch auswerten zu können, ist ein phasenempfindlicher Verstärker nachgeschaltet. Die Probeentnahme aus der Flasche erfolgt dabei im Zwischenstern, zweckmäßig vor dem Prüfkreisel des Massenspektrometers.

Über der Flaschenmündung befindet sich eine Düse, mit der das Probegas aus der Mehrwegflasche geblasen und von dem Analyse-Gerät durch eine Vakuumpumpe übernommen wird. Wird eine kontaminierte Flasche erkannt, erfolgt die Ausschleusung über einen anschließenden Vakuumstern auf das erste Ausschleusband, wo sie dann mittels bekannter Einrichtungen vom Band entfernt wird.

Um nun weitere andere Stoffe auszuschleusen, kann das Photoionisations-Verfahren zur Anwendung gelangen. Dieses ist geeignet zur Erkennung von organischen Stoffen, wie Öle sowie anorganischen Gasspuren, wie Schwefelwasserstoff, Ammoniak und dgl. Ferner sind mit diesem Verfahren Verbindungen mit Chlor, Fluor, Brom und Jod in bestimmten Funktionsgruppen gut nachweisbar. Auch kann das Probenahmesystem mit einer Flammionisationsanalyse ausgestattet sein. Dieses Verfahren besteht darin, daß die Ladungsträger in einer chemischen Reaktion beim Einleiten des Probegases in einer Wasserstoffflamme erzeugt werden. Im Gegensatz zum PID werden keine organischen Stoffe sondern alle organischen Luftinhaltsstoffe mit CH-Verbindung gemessen. Die Kombination von FID und PID ermöglicht dabei eine erste Stoffklassifizierung, wobei dieses Verfahren ergänzend zum PID eingesetzt werden kann und zum Aussortieren von Flaschen dient, in denen andere Inhaltsstoffe als Mineralwasser waren. Die jeweilige Detektionsschwelle des Verunreinigungsgrades ist bei diesen Verfahren einstellbar. Wenn alle Flaschen, in denen eine andere Flüssigkeit als Mineralwasser enthalten war, selektiert werden sollen, steht die jeweilige Einstellung auf der empfindlichsten Stufe. Die als kontaminiert erkannten Flaschen werden dann ausgeschleust und beispielsweise wieder in Behälter gesetzt. Sie können der Anlage erneut zugeführt werden, wenn beispielsweise Limonaden oder dgl. abgefüllt werden.

Beim Abfüllen von Limonaden wird wiederum der PID zur Vorselektion stark kontaminierter Flaschen eingesetzt.

Im Anschluß daran wird die eigentliche Analyse der Mehrwegflaschen mit dem Massenspektrometer durchgeführt, wobei beispielsweise die vorsortierten Flaschen über einen Stern dem eigentlichen Prüfkreisel zugeführt, im Mündungsbereich durch spezielle Zangengreifer erfaßt und mittels Hubkurve angehoben werden. Dabei taucht ein Saugrohr in die Behältermündung und zieht Meßgas aus dem Inneren der Flasche. Dieses Meßgas wird von einer Vakuumpumpe über einen Drehschieber kontinuierlich abgesaugt. Ein Teilstrom gelangt dann in die Ionisationskammer des Massenspektrometers.

## Patentansprüche

1. Verfahren zur Bestimmung von kontaminierten Mehrwegflaschen oder Behältern mittels verschiedenartiger Analysatoren in einem Probenahmesystem, **dadurch gekennzeichnet,** daß ein erstes Probenahmesystem angeordnet ist, dessen zugeführte Proben einer Stickstoffoxyd-Analyse unterworfen werden und ein zweites Probenahmesystem vorgesehen ist, dessen Proben einer Fotoionisationsanalyse und/oder Flammionisationsanalyse unterworfen und die vorgeprüften Flaschen dann einer Massenspektrometeranalyse unterworfen werden.

2. Verfahren zur Bestimmung von kontaminierten Mehrwegflaschen gemäß Oberbegriff des Anspruchs 1, **dadurch gekennzeichnet,** daß die zugeführten Flaschen unmittelbar vor der eigentlichen Hauptanalyse einer ein- oder mehrfachen Voranalyse unterworfen, die dabei als fehlerhaft oder ungeeignet erkannten Flaschen aussortiert und die ordnungsgemäßen Flaschen einer weiteren Analyse unterworfen werden.

3. Verfahren zur Bestimmung von kontaminierten Mehrwegflaschen gemäß Oberbegriff Anspruch 1, **dadurch gekennzeichnet,** daß eine erste Analyse vorgenommen wird, bei welcher die Flaschen ermittelt und ausgeschleust werden, in denen eine andere als die zur jeweiligen Füllung vorgegebene Flüssigkeit enthalten war und diese Flaschen erneut zugeführt werden, wenn die in diesen analysierte Flüssigkeit zur Abfüllung gelangt.

4. Verfahren nach den Ansprüchen 1 - 3, **dadurch gekennzeichnet,** daß die Inspektion kontaminierter Behälter mit mehreren Detektoren an einem zentralen, stationären und dichtungslosen Probenahmesystem erfolgt, unter dem die Behälter mittels einer Transportvorrichtung vorbeigeführt werden.

5. Verfahren nach den vorhergehenden Ansprüchen, **dadurch gekennzeichnet,** daß die Probe mittels einer Cross-Flow modulierten Meßgasaufbereitung erfolgt.

6. Verfahren nach den vorhergehenden Ansprüchen, **dadurch gekennzeichnet,** daß durch den kombinierten Einsatz einfacher Summendetektoren, wie Hableitersensor, FID, PID, ICD und/oder anderen zum einen der Vielzahl von möglichen Kontaminaten Rechnung getragen wird, wobei aus der Ergebnismatrix Rückschlüsse auf natürliche Kontaminate der Behälterfüllung geschlossen werden.

7. Verfahren nach den vorhergehenden Ansprüchen, **dadurch gekennzeichnet,** daß ein und derselbe Summendetektor mit und ohne vorgeschalteten Katalysator mehrfach zur Anwendung gelangt.
